# EUROPEAN PATENT APPLICATION

(11) **EP 2 586 396 A1**
(43) Date of publication of application: **01.05.2013**
(21) Application number: 11186694.3
(22) Date of filing: 26.10.2011
(51) Int. Cl.: A61B 19/00

(54) **System for ensuring precision in medical treatment**

(71) Applicant: Metronor AS, 1394 Nesbru (NO)
(72) Inventor: Oyvind, Rotvold, 1395 Hvalstad (NO)
(74) Representative: Lang, Johannes

(57) **Abstract**

A system for ensuring precision in medical treatment of a patient comprising
- one or more clusters of targets (11) attached to a patient (2), a strapping mechanism or a carrier (3) defining a patient-fixed coordinate system (2a),
- a first electro-optical measurement system for observing the one or more clusters of targets (11) and local reference markers (5) of an imaging system (4) to determine the relationship between the patient-fixed coordinate system (2a) and a coordinate system (5a) of the imaging system (4), whereby a location (1) to be treated inside the patient (2) can be calculated in the patient-fixed coordinate system (2a), and
- a second electro-optical measurement system for observing the one or more clusters of targets (11) and local reference markers (9) of a treatment system (8), whereby the location (1) to be treated inside the patient (2) can be calculated in a coordinate system (9a) of a treatment system (8).

## Description

### Field of use - background

Advances in modern medical technology enable new treatments that have the potential for higher geometrical precision and more localized effects than was previously possible, avoiding damage to surrounding organs and tissues.

For example, it is now possible to irradiate cancerous tumors more precisely than with legacy treatment methods that irradiate more widely and therefore potentially damage large volumes of healthy organs and tissue. Particularly for young patients, such advances dramatically lower the probability of developing cancer later in life as a consequence of the radiation treatment, but also for older patients improved precision enables higher radiation doses for the cancerous cells without increased risk of damaging surrounding healthy organs and tissue.

As a specific example, proton ray oncology treatment is increasingly replacing older radiation treatments due to the unique absorption characteristics of proton rays.

Older radiation methods send harmful rays through the patient's body, causing damage along the entire entry and exit paths as well as at the tumor itself. The tumor is therefore irradiated from a wide range of angles to distribute entry and exit path radiation over a large volume of organs and tissue in order to keep radiation dosage per volume as low as possible for the surrounding organs and tissues. Since there is therefore a strong relationship between intentional irradiation of the tumor and potentially very dangerous consequential irradiation of healthy organs and tissue, this limits the radiation dosage that can be administered on the tumor and subjects the patient to significant risk of damage to healthy organs and tissue - increasing discomfort during and immediately following treatment and increasing the likelihood of developing cancer later in life as a consequence of the treatment.

Proton rays have been found to have two important characteristics that make them very well suited for radiation treatment systems. First, due to heavy particle mass, proton rays have low scatter so can be more precisely aimed through organs and tissue. Second, proton rays can be energized more or less highly to control how far into the body they will penetrate, and they deposit the majority of their energy within the last few millimeters of penetration (the so-called Bragg peak). The radiation dose can therefore be targeted in a more closely defined area than for conventional radiation treatment, and minimizes damage along the entry path and virtually eliminates damage beyond the targeted tumor. This enables higher radiation dosage for the tumor for more efficient treatment combined with significantly reduced radiation dosage for surrounding healthy organs and tissue and consequently lower probabilities for developing cancer later in life.

Proton ray treatment systems are extremely expensive, currently typically costing 100 ― 300 million dollars to build. To justify the cost, it is therefore necessary to ensure that the benefit from such systems is maximized.

To take full advantage of such new technologies, it is necessary to dramatically increase the geometrical precision of the treatment. For example, it is necessary to precisely determine the size and location of the tumor inside the patient, and to transfer and utilize this information from the imaging systems to the radiation treatment equipment without loss of precision in order to ensure that the radiation is deposited exactly at the tumor's location. With increased radiation dosages and well-defined absorption depth, it is clearly critical that the radiation beam is positioned and oriented correctly relative to the tumor. Industry states a goal of equipment precision of 0.1mm in all three axes.

Increased precision is of course also of benefit in older treatment methods, as a better precision permits administering the best-possible treatment precision given the inherent limitations of the radiation or other treatment technique itself.

### State of the art

Various imaging technologies are used to determine the size and location of areas needing treatment such as e.g. a tumor; such technologies include X-ray, Magnetic Resonance Imaging (MRI) or Computed Tomography (CT) scanning. These systems provide information on the size, shape and location of the area needing treatment relative to a local imaging system-specific coordinate system.

Many imaging systems are susceptible to damage if subjected to radiation and must therefore be located away from the radiation systems.

For treatment, the treatment system must then position the patient correctly relative to the treatment tool - e.g. the radiation source - for the tumor to receive maximum radiation dose while ensuring minimal damage to surrounding organs and tissue. To further reduce damage and to account for the shape of the tumor, irradiation is often performed from a range of directions and at different times. This makes the positioning of the tumor relative to the radiation equipment a repetitive process.

Patient geometry is commonly stabilized by mechanically connecting the patient - or the relevant parts of a patient's body - to a mechanical carrier using various strapping systems, casts, molds, fixing systems or even screws.

Currently, all imaging and treatment instruments - even if located in widely separated parts of a facility - are installed in a common master coordinate system, often referred to as an 'ISO system'. This common master coordinate system is represented by physical markers, often referred to as fiducials, located throughout the facility. The relative positions of the entire network of markers in relationship to the common master coordinate system are measured using precision techniques such as theodolites or laser trackers.

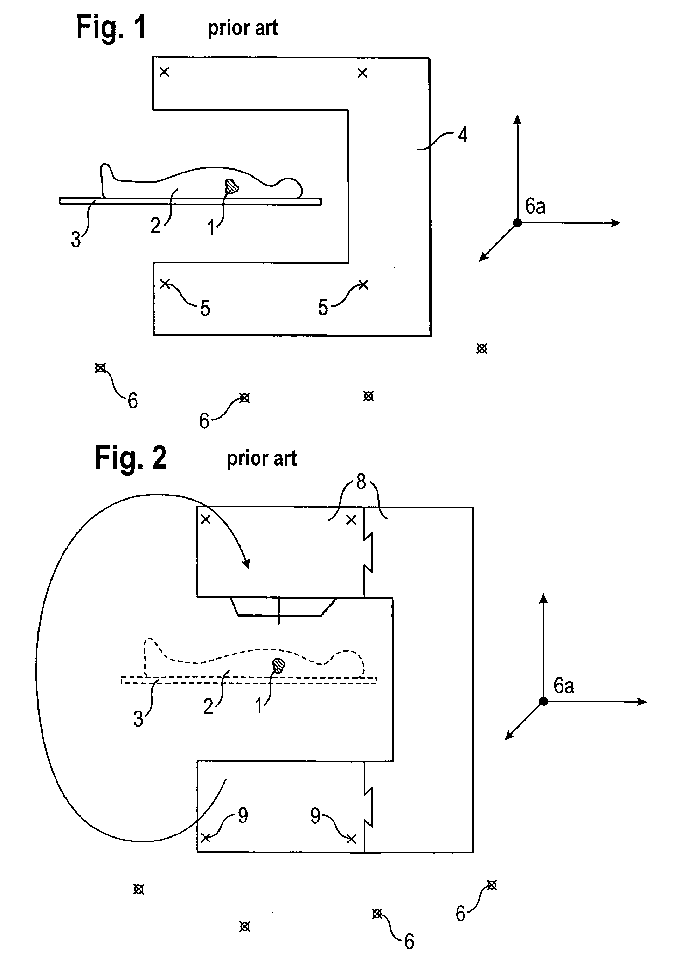

This is shown in schematic Figure 1, where position of a tumor 1 inside a patient 2 on a carrier 3 is determined by an imaging system 4 having local reference markers 5 precisely known in relation to the common master coordinate system 6a represented by the network of reference markers 6.

As shown in schematic Figure 2, a treatment system 8 also has local reference markers 9 precisely aligned with the common master coordinate system 6a represented by the network of reference markers 6. For practical reasons, the network of reference markers must therefore extend throughout the facility to enable the precise determination of all markers relative to the common master coordinate system 6a.

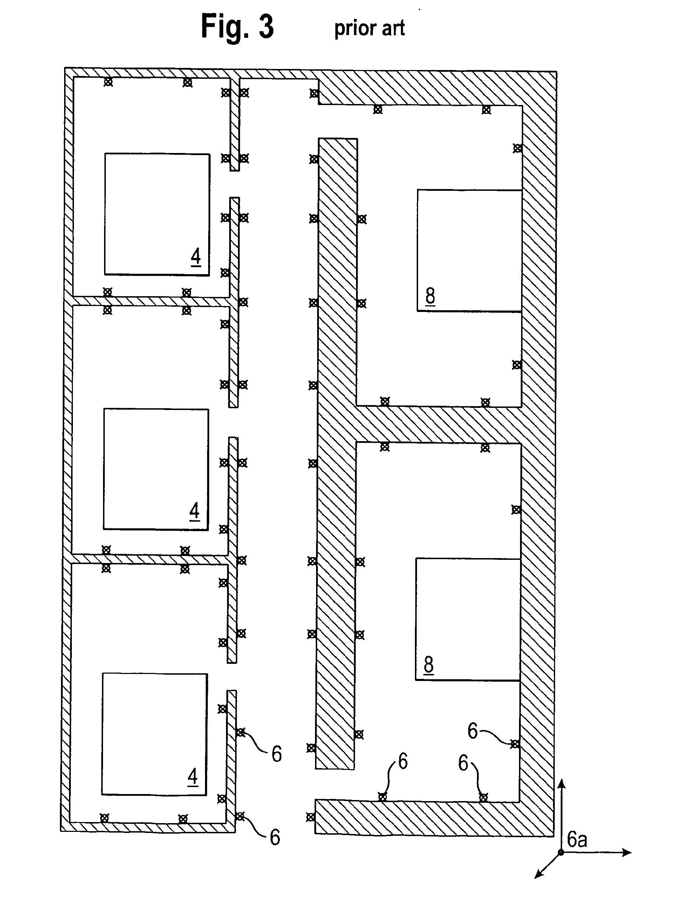

Schematic Figure 3 shows an example of a portion of a facility, and specifically how the network of reference markers 6 extend from the locations of the imaging systems 4 to the locations of the treatment systems 8 in order to establish a common master coordinate system 6a.

It is a well-known problem that establishing a network of reference markers is a time-consuming and difficult task, particularly when no line-of-sight is available between the various rooms of a larger facility. There typically will also be a need for markers in areas of no particular interest such as corridors, simply because they are needed to enable the measurement instruments to tie the various areas of actual interest together. To this comes the added difficulty of buildings being only nominally stable - even very heavy steel and concrete buildings easily deform by many times the stated goal of 0.1mm in response to everyday thermal and mechanical loads.

Returning to the proton treatment example for illustration, the proton gun/nozzle assembly is typically a massive construction weighing many tons. In many cases, the gun/nozzle is rotatable around the patient - as shown in Figure 2 ― and deformation of the rotatable structure of several millimeters is common and must typically be compensated for at frequent intervals.

The purpose of the common master coordinate system is to ensure that location data acquired with an imaging system can be transferred to and used by a treatment system, such as e.g. a radiation system. Therefore, any measurement errors made when establishing the network, or any instability in the network itself, or any misalignment made when installing either system, will impact the precision with which geometry information is transferred between the imaging systems and the radiation systems. It is clear that the current process is unsuited to support a requirement for 0.1 mm accuracy throughout a large facility and over time.

In addition to the need to mechanically align all units within the common master coordinate system, it is under the current state of the art also necessary to precisely predict any deviation within each unit's local coordinate system. For example, since the patient carrier is maneuvered in the common master coordinate system it is not only necessary to know its exact installed position and orientation, but any inaccuracy throughout its range of travel must also be understood. Essentially a robot, the carrier system must therefore be precisely calibrated and the calibration data may need to take into account variable parameters such as patient weight, patient weight distribution and the carriers angle to gravity. Similarly, the characteristics of a rotating gun/nozzle assembly needs to be precisely understood and compensated for as any shift in position or direction of the unit is clearly undesirable - due to its immense weight, the rotating gun/nozzle assembly may display significant deformations through its arc of travel. Achieving compensation values able to support a 0.1 mm accuracy requirement over time and other influences is clearly extremely challenging.

The state of the art therefore incurs significant efforts and costs yet is ill suited to support the stated accuracy requirements.

### Present invention

The present invention involves a novel approach to acquiring and transferring the critical geometrical information between imaging and treatment systems with minimal error stack-up. The present invention completely eliminates the need for a common master coordinate system throughout the facility, provides accuracy independent of facility size and independent of the individual placement of the various imaging and treatment systems and does not rely on a stable facility structure. The present invention eliminates the effects of most error contributions in the current process, eliminates the need for aligning systems to the common master coordinate system during installation, and also eliminates the need for mapping and calibration of the characteristics of several system elements.

These objectives are achieved according to the invention by a system as defined in claim 1 or 2. Advantageous embodiments of this system are defined in dependent claims 3 to 15.

The invention therefore provides for higher treatment precision while reducing the cost and complexity of establishing and maintaining networks of reference markers.

The present invention introduces a patient-fixed coordinate system. The patient - or the relevant parts of the patient's body - is typically strapped to a carrier to ensure stability during imaging and treatment. By attaching one or more clusters of targets to the patient, the strapping mechanism or the carrier - as long as these are in stable positions relative to each other it is not important which embodiment is used - any information known relative to the patient will also be known relative to the one or more clusters of targets.

If the targets are suitable for observation by an electro-optical measurement system such as those described e.g. in EP 0 700 506 B1, EP 0 880 674 B1, EP 0 829 701 B1, EP 1015 845 B1 and EP 1447 644 A1. The electro-optical system can easily and with very high precision determine the position of the one or more clusters of targets relative to its own coordinate system. As the imaging systems also have local reference markers-currently used to align it into the 'ISO center' or common master coordinate system - the electro-optical measurement system can also determine the location of these local reference markers in its own coordinate system. If the local reference markers are not suitable for direct observation by the electro-optical measurement system, then the use of e.g. a probing device such as that described in EP 0 607 303 B1 may be used.

Now, if the electro-optical measurement system observes both the one or more clusters of targets attached to the patient, the strapping mechanism or the carrier and the local reference markers of the imaging system, then the relationship between the one or more clusters of targets, i.e. the patient-fixed coordinate system and the coordinate system of the imaging system can be determined.

The imaging system determines the locations to be treated inside the patient in reference to the imaging system's internal coordinate system. The locations to be treated inside the patient will be reported in this internal coordinate system of the imaging system, but by using the measured relationship between the imaging systems' coordinate system and that of the patient-fixed coordinate system, it is now possible to calculate the locations to be treated inside the patient in the patient-fixed coordinate system.

The patient can now be moved any distance from the imaging system, and the information about the locations to be treated inside the patient in the patient-fixed coordinate system will be retained without loss of accuracy.

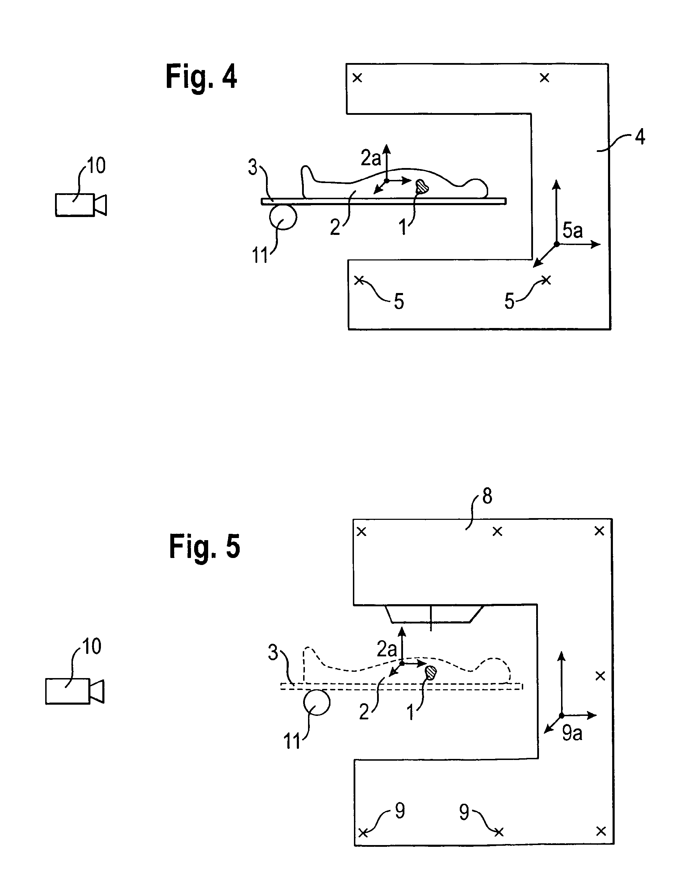

The principle is shown in schematic Figure 4, where the tumor 1 inside the patient 2 on the carrier 3 is determined by the imaging system 4 having a local coordinate system 5a represented by the local reference markers 5. Notice that the local reference markers 5 only relate to the imaging system itself; they need not be known relative to any other unit or system. This significantly simplifies installation and replacement of the imaging system. The electro-optical measurement system consists of a sensor 10 observing one or more clusters of targets 11 in known positions relative to the patient 2 or the carrier 3, as well as the local reference markers 5. It is therefore possible to relate information about the tumor 1 location acquired relative to the local reference markers 5 to the patient 2 or the carrier 3, i.e. the patient-fixed coordinate system.

Turning now to schematic Figure 5, according to the present invention the treatment system 8 also has local reference markers 9 that are in known positions relative to the treatment system and independent of any external unit or system. The sensor 10 of the electro-optical measurement system observes these local reference markers 9 as well as the clusters of targets 11 in known positions relative to the patient 2 or carrier 3. Therefore, the location of the tumor 1 can be calculated relative to the local reference markers 9 of the treatment system and therefore the treatment system's local coordinate system 9a.

The main effects of the present invention are:
1 ― elimination of the costs and efforts related to establishing and maintaining the common master coordinate system
2 ― significant reduction in the need to calibrate parts of the total system
3 ― elimination of all error sources related to network imprecision, system misalignment and network instability over time and external influences
4 ― improved precision in transferring geometry information on the location to be treated inside the patient from the imaging system to the treatment system
5 ― ability to provide closed-loop feedback for guiding the patient relative to the treatment system independent of e.g. carrier calibration accuracy.

The present invention therefore introduces significant improvements in operation of medical treatment systems as described, and contributes significantly to realizing their potential benefit. Given the extreme costs of certain such systems, the present invention is an important contribution towards making these systems viable to a larger population of patients.

### Details of the invention

The imaging system can be any type of diagnostic system able to provide data useful for determining the spatial locations of areas to be treated inside a patient. Typical examples include X-ray, MRI and CT scanning systems.

The electro-optical system will typically consist of three types of modules - one or more sensors, a set of targets or a mechanism with targets to acquire the markers, and a computer with software to extract the spatial positions of the targets.

The targets and markers can be of a variety of types - passive, active or mechanical.

Passive targets would include any 2D geometrical figure suitable for observation such as e.g. cross-hairs, squares, circles or other regular geometrical shapes or combinations of such. Passive targets could be marks on a normal substrate like paper, plastic film or the like, or they could be retro-reflective to optimize the return of light energy. Such targets could be illuminated by a light-source synchronized with the sensor or by general background lighting.

Passive targets could also, depending on the sensor system, include 3D physical shapes suitable for spatial determination of a characteristic point such as e.g. the center of spheres, a corner of a cube or other polyhedron, or any other regular or well-defined body.

Active targets may be advantageous to ensure optimal signal-to-noise ratios. Active targets include Light Emitting Diodes, laser sources, and incandescent sources. They may be synchronized with the sensor exposure, have timing and intensity adjusted automatically based on sensor based on the observations made, have a fixed cycle rate or be constantly illuminated.

Active targets may contain passive elements, e.g. with a back-lit translucent target surface with a passive target pattern engraved, printed or otherwise placed on the surface. Active targets can also be indirect whereby an active light source illuminates a passive surface to create a projected target.

Targets can also be of a mechanical type that is not directly observable by the sensor, or they could be in a location not directly observable from the sensor's position. In such cases, the targets position can be acquired by the use of a probe with observable targets embedded into it, as described e.g. in EP 607 303 B1 or EP o 880 674 B1.

The shape and size of the clusters of targets can be designed to fit the specific application in order to ensure that the targets are viewable by the sensor or sensors, do not interfere with the movement of the other system elements and provide sufficient target spacing to achieve the required accuracy.

It may be advantageous to provide the one or more clusters of targets 11 with high-precision attachment mechanism so that it can be released from the carrier or the patient and re-attached without appreciable loss of precision. Several suitable mechanisms are commercially available, e.g. for attaching navigation equipment to aircraft, or probes to conventional coordinate measurement systems.

This would enable the use of different types or shapes of clusters of targets for each type of imaging or treatment system as long as the cluster attachment mechanism has sufficient precision and the various types or shapes of clusters of targets all represent the same coordinate system relative to the attachment mechanism. This may be practical e.g. if an imaging system limits the shape of the one or more clusters of targets in one dimension, while a treatment system may limit the shape or size in another direction.

The electro-optical system sensor can also be of a number of different types.

Cameras with a 2D sensor, such as commonly used in digital still or video cameras can be used. An alternative would be 1D array sensors and conventional or cylindrical optics oriented with two sensors vertically for triangulation in azimuth and one horizontal for determination of elevation to the targets. It is also possible to use a combination of 1D and 2D sensors.

Another alternative is to use automated theodolites. These instruments incorporate a 2D camera as well as servo motors to point the camera in precisely known directions, alternatively with feed-back from image analysis to home into specific targets.

It is well known e.g. from EP 0 880 674 B1 that a combination of 2D cameras and a laser rangefinder can produce an increased working volume and improved accuracy along the camera optical axis compared to just using a camera, and various implementations are commercially available. Since such systems are also capable of determining the position of a cluster of targets relative to a set of references, they can also be used. An example of a cluster of targets suitable if such a sensor is selected is e.g. the T-Mac™ product marketed by Leica Geosystems™.

Yet another possible electro-optical sensor configuration would be similar in principle to the IntelliProbe 360™ marketed by Automated Precision, Inc. This type of sensor combines a laser tracker tracking a conventional spherically mounted retro-reflector or other target. Since this only yields a single point and therefore cannot determine the orientation of a target in space, a set of orientation sensors is added to acquire unit orientation. Orientation sensors may include gyro instruments, tilt sensors or Hall-effect sensors.

It is also known, e.g. from EP 1015 842 B1 that the reverse configuration can be used - an electro-optical sensor may be positioned in a known relationship to the patient or the carrier (where the cluster of targets would otherwise be placed), while a cluster of targets may be placed in a suitable, stable position (e.g. where the sensor would otherwise be placed).

### Practical embodiments of the invention

The local reference markers 5 in Figure 4 and the local reference markers 9 in Figure 5 may not be provided by the manufacturers of the imaging and treatment system.

In such cases, they can for imaging systems be created e.g. by positioning local reference markers inside the working envelope of the imaging system that can be imaged by the imaging system and also measured by the optical measurement system so that the relationship between the electro-optical measurement system and the coordinate system of the imaging system can be determined. Alternatively, more complex markers could be used that combine markers suitable for the imaging system and markers for the electro-optical measurement system and located in known positions relative to each other. In both cases, a single marker can be moved to multiple positions, or multiple markers can be measured and imaged by both systems.

Once the relationship between the electro-optical measurement system and the coordinate system of the imaging system has been determined, reference markers of the preferred type can be positioned in suitable locations on or about the imaging system and their location relative to the coordinate system of the imaging system can be determined by the electro-optical measurement system. This enables placement of markers that are suitable for repeated use by the electro-optical system and for practical reasons these markers will typically be placed outside the imaging system's working envelope.

Alternatively, the relationship between the coordinate system 5a of the imaging system 4, and the position and orientation of the electro-optical measurement system - represented by the sensor 10 ― can be used in lieu of any local reference markers 5. Given that the relationship between the coordinate system of the imaging system and the electro-optical measurement system has been established - e.g. as described above - and as long as the sensor of the electro-optical system 10 is stable relative to the imaging system 4, this relationship therefore makes it possible to directly determine the locations to be treated inside the patient 2 from measurement of the one or more clusters of targets 11 in known positions relative to the patient 2 or the carrier 3; i.e. the patient-fixed coordinate system without any local reference markers 5.

Regardless of whether local reference markers are available, are created or the electro-optical system's relative position is used, the present invention provides a means of transferring the location of the tumor inside the patient - which was determined in the imaging system's coordinate system - to a coordinate system known relative to the patient or the carrier, i.e. to the patient-fixed coordinate system.

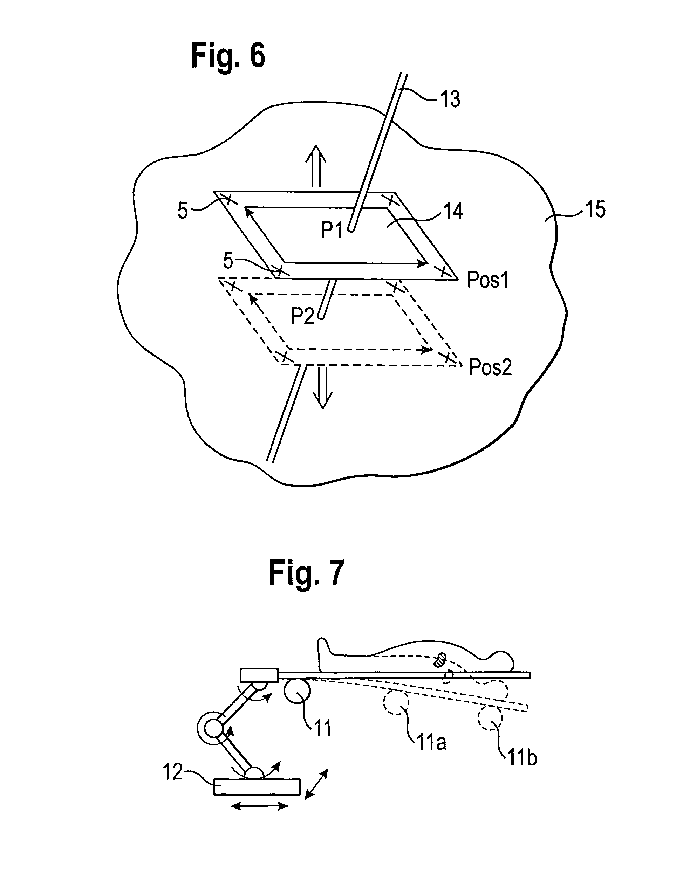

For treatment systems, procedures typically exist to determine the precise location - position and direction - of the radiation beam in space as a function of the treatment center's configuration with respect to e.g. nozzle rotation. This is typically done with a 2D imaging radiation sensor 14 as shown in schematic Figure 6. Using e.g. local reference markers 9 measured by the electro-optical measurement system, the position and orientation of the radiation sensor 14 is known. The radiation sensor 14 registers the center of the radiation beam 13 inside the two-dimensional sensor surface. For one radiation sensor position - Pos 1 in Figure 6 - one point P1 along the radiation beam can thus be determined. By moving the sensor 14 along a suitable spatial direction to e.g. Pos 2 in Figure 6 second point P2 along the radiation beam 13 can be determined, and therefore the direction of the beam 13 for this configuration.

The radiation deposited e.g. by protons varies strongly with penetration depth into tissue, and also may need to be determined. If the radiation sensor 14 is placed inside a suitable material 15 with properties similar to those of human tissue, such as water, one may monitor the relative strengths of the radiation reading in P1, P2 and additional readings taken in additional radiation sensor positions, this characteristic can be measured. In such a case, it is more practical to place the local reference markers 9 on the outside of the container for the material 15 and to use a separate measurement device such as linear displacement meters to determine the position of the radiation sensor 14 inside the material 15.

The present invention therefore captures and accurately transfers the required spatial information from the imaging system to the treatment system, where the information can be used to position the treatment system and the patient in the appropriate relative positions.

The electro-optical measurement system can be used to continuously or at given intervals measure the actual relative positions between the treatment system and the one or more clusters of targets, and thereby the patient. These measurements can be used to verify the positioning and thus prevent erroneous or inaccurate treatment, or they can be provided as closed-loop input to the systems positioning the patient relative to the treatment system.

While some treatment systems are stationary, so that the patient must be moved relative to the treatment system, typically by a robotic mechanism 12 such as that shown in schematic Figure 7, others are movable - or have relevant parts that are moveable - in one or more axes.

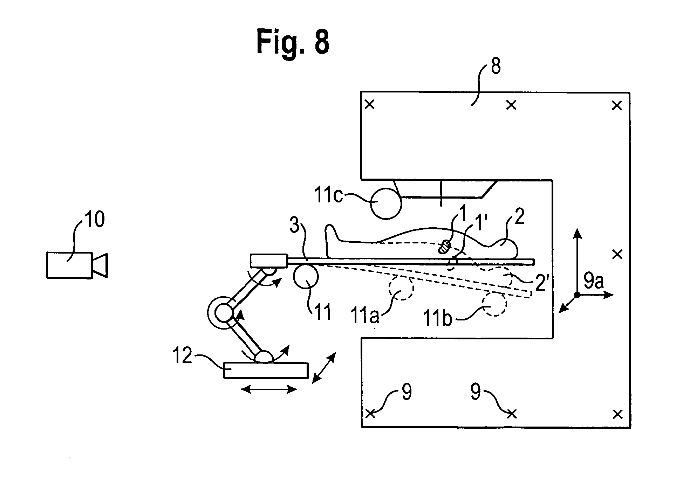

For movable systems, the present invention provides for a method for tracking the relevant parts of the treatment system, as shown in schematic Figure 8. In such a case, one or more clusters of targets 11c would be placed in a known position relative to the moveable part of the treatment system, and by measuring the position of these one or more clusters of targets 11c and the one or more clusters of targets 11, 11a, 11b connected to the patient, their relative positions can be determined as they move relative to each other. In this embodiment, the present invention can be used to compensate for inaccuracies caused both by treatment system deformations as well as carrier deformations and robotic system inaccuracies, thus significantly reducing the need for time-consuming equipment calibration procedures.

Notice that the electro-optical system used with the imaging system may, but need not, be the same as the one used with the treatment system. In certain embodiments, using a single, portable system may be advantageous. In most embodiments, however, it is likely more advantageous to use different, more permanently installed electro-optical systems with each imaging and treatment systems.

Notice also that the relationship between the patient, or the part of the patient's body needing treatment, and the carrier or strapping mechanism may not be entirely stiff and could therefore introduce an error source. An example of such an error source would be the mechanical bending of a carrier under the load of the patient as shown in Figure 7. Any such bending would change with the angle of the carrier relative to the direction of gravity, and unless compensated for the variation in bending between the imaging position and the treatment position would cause an error several times the precision goal.

Compensation could be introduced in several ways. For example, the electro-optical system could be used to determine the angle between gravity and the carrier and a calibration table based on empirical experimental data could be used to estimate bending as a function of load, load distribution and angle. Alternatively, one or more clusters of targets 11a, 11b could be placed along the carrier to directly determine the amount of bending using the electro-optical measurement system.

The bending would also depend on the way the carrier is attached to the treatment or imaging system, and it would be advantageous if the same attachment locations on the carrier were always used. Alternatively, methods similar to those outlined above could be used.

Notice, however, that is the patient is held the same way in the imaging and treatment systems and the angle relative to gravity is the same, then any bending of the carrier will with the present invention be the same in both cases and does not need to be compensated for, regardless of patient weight and position.

### Reference numeral list

- 1: Tumor
- 2: Patient
- 2a: Patient-fixed coordinate system
- 3: Carrier
- 4: Imaging system
- 5: Local reference marker
- 5a: Local coordinate system of the imaging system
- 6: Reference marker
- 6a: Common master coordinate system
- 8: Treatment system
- 9: Local reference marker
- 9a: Local coordinate system of the treatment system
- 10: Sensor
- 11, 11a, 11b, 11c: Cluster of targets
- 12: Robotic mechanism
- 13: Beam
- 14: Radiation sensor
- 15: Material

## Claims

1. System for ensuring precision in medical treatment of a patient, particularly for the treatment of cancerous tumors by proton rays comprising:
- a carrier (3) for the patient (2),
- a strapping mechanism for ensuring stability of the patient (2) on the carrier (3) during imaging and treatment,
- an imaging system (4) for determining the location (1) to be treated inside the patient (2), said imaging system (4) comprising local reference markers (5) in locations known relative to the coordinate system (5a) of the imaging system (4),
- a treatment system (8) comprising also local reference markers (9) in locations known relative to the coordinate system (9a) of the treatment system (8),
**characterized by**
- one or more clusters of targets (11) attached to the patient (2), the strapping mechanism or the carrier (3) defining a patient-fixed coordinate system (2a),
- a first electro-optical measurement system for observing the one or more clusters of targets (11) and the local reference markers (5) of the imaging system (4) to determine the relationship between the patient-fixed coordinate system (2a) and the coordinate system (5a) of the imaging system (4), whereby the location (1) to be treated inside the patient (2) can be calculated in the patient-fixed coordinate system (2a),
- a second electro-optical measurement system for observing the one or more clusters of targets (11) and the local reference markers (9) of the treatment system (8), whereby the location (1) to be treated inside the patient (2) can be calculated in the coordinate system (9a) of the treatment system (8).

2. System for ensuring precision in medical treatment of a patient, particularly for the treatment of cancerous tumors by proton rays comprising:
- a carrier (3) for the patient (2),
- a strapping mechanism for ensuring stability of the patient (2) on the carrier (3) during imaging and treatment,
- an imaging system (4) for determining the location (1) to be treated inside the patient (2),
- a treatment system (8),
**characterized by**
- one or more clusters of targets (11) attached to the patient (2), the strapping mechanism or the carrier (3) defining a patient-fixed coordinate system (2a),
- a first electro-optical measurement system for observing the one or more clusters of targets (11) and for establishing the relationship between the coordinate system (5a) of the imaging system (4) and the position and orientation of the first electro-optical measurement system to determine the relationship between the patient-fixed coordinate system (2a) and the coordinate system (5a) of the imaging system (4), whereby the location (1) to be treated inside the patient (2) can be calculated in the patient-fixed coordinate system (2a),
- a second electro-optical measurement system for observing the one or more clusters of targets (11) and for establishing the relationship between the coordinate system (9a) of the treatment system (8) and the position and orientation of the second electro-optical measurement system, whereby the location (1) to be treated inside the patient (2) can be calculated in the coordinate system (9a) of the treatment system (8).

3. System according to claim 1 or 2, wherein the first and second electro-optical system is a single portable system.

4. System according to one of claims 1 to 3, wherein the electro-optical system comprises the three types of modules, namely one or more optical sensors (10), the one or more clusters of targets (11) attached to the patient (2), the strapping mechanism or the carrier (3) and a computer with software
- to determine the relationship between the patient-fixed coordinate system (2a) and the coordinate system (5a) of the imaging system (4), whereby the location (1) to be treated inside the patient (2) can be calculated in the patient-fixed coordinate system (2a), and
- to calculate the location (1) to be treated inside the patient (2) in the coordinate system (9a) of the treatment system (8) by means of the calculated location (1) to be treated inside the patient (2) in the patient-fixed coordinate system (2a).

5. System according to one of claims 1 to 4, wherein the one or more clusters of targets (11) are attached to the patient (2), the strapping mechanism or the carrier (3) with a high-precision attachment mechanism, so that it can be released and reattached without appreciable loss of precision.

6. System according to claim 5, wherein different types or shapes of clusters of targets are usable for each type of imaging system and treatment system, whereby the different types or shapes of clusters of targets represent the same coordinate system relative to the high-precision attachment mechanism.

7. System according to one of claims 1 to 6, wherein the targets (11) respectively the local reference markers (5, 9) are passive, active or mechanical targets respectively local reference markers (5, 9).

8. System according to one of claims 3 to 7, wherein the optical sensor comprises any of the following:
- 2D imaging sensor,
- 2D imaging sensor combined with encoders to precisely determine the orientation of the 2D imaging sensor,
- a combination of 1D (array) imaging sensors in known spatial relations
- a laser rangefinder and a 2D imaging sensor,
- a laser rangefinder and a combination of 1D (array) imaging sensors in known spatial relations,
- a laser rangefinder combined with one or more clusters of targets that also contains orientation sensors to determine the orientation of the cluster.

9. System according to claim 1 or one of claims 3 to 8, wherein the local reference markers (5) of the imaging system (4) are positioned inside the working envelope of the imaging system (4) that can be imaged by the imaging system (4) and also measured by the first electro-optical measurement system.

10. System according to claim 1 or one of claims 3 to 8 comprising more complex markers combining markers suitable for the imaging system and markers suitable for the electro-optical measurement system and located in known position relative to each other.

11. System according to claim 9 or 10, comprising a single marker movable to multiple positions.

12. System according to one of claims 3 to 11, wherein at least parts of the treatment system (8) are movable and wherein one or more clusters of targets (11c) are placed in known positions relative to the movable parts of the treatment system (8) to determine the positions of the one or more clusters (11c) placed in known positions to the movable parts of the treatment system relative to the positions of the one or more clusters (11, 11a, 11b) attached to the patient (2), the strapping mechanism or the carrier (3).

13. System according to one of claims 1 to 12, wherein the electro-optical measurement system is suitable to determine the angle between gravity and the carrier (3), whereby bending of the carrier (3) as a function of load, load distribution and angle is estimated based on a calculation table resulting from empirical data.

14. System according to one of claims 1 to 12, comprising one or more clusters of targets (11, 11a, 11b) placed along the carrier (3) to directly determine the amount of bending using the electro-optical measurement system.

15. System according to one of claims 1 to 14, wherein the imaging system (4) includes an X-ray imaging system, a magnetic resonance imaging system or a computer tomography scanning system.
